# EUROPÄISCHE PATENTANMELDUNG

(11) **EP 0 785 254 A2**
(43) Veröffentlichungstag der Anmeldung: **23.07.1997**
(21) Anmeldenummer: 97100216.7
(22) Anmeldetag: 09.01.1997
(51) Int. Cl.: C12N 15/12, C12N 15/54, C07K 14/705, C12N 9/12, C12N 15/11, A61K 31/70

(54) **Phosphatidyl-Inositol-Glucal Klasse F (PIG-F), die dafür kodierende cDNA und deren Verwendung als Arzneimittel**

(30) Priorität: 18.01.1996 DE 19601628
(71) Anmelder: BAYER AG, 51368 Leverkusen (DE)
(72) Erfinder: Rübsamen-Waigmann, Helga, Prof.Dr., 65812 Bad Soden (DE); Selders, Gottfried, 63069 Offenbach (DE); Strebhardt, Klaus, Dr., 60489 Frankfurt (DE)

(57) **Zusammenfassung**

Die vorliegende Erfindung betrifft PIG F-cDNA und deren Verwendung als Arzneimittel. Diese DNA ist in antisense Orientierung geeignet zur Bekämpfung von Tumoren.

SEQUENZPROTOKOLL

(1) ALLGEMEINE ANGABEN:

(i) ANMELDER:
(A) NAME: Bayer AG
(B) STRASSE: Bayerwerk
(C) ORT: Leverkusen
(E) LAND: Deutschland
(F) POSTLEITZAHL: 51368
(G) TELEFON: 0214-3061455
(H) TELEFAX: 0214-303482

(ii) BEZEICHNUNG DER ERFINDUNG: PIG-F, die dafür kodierende c-
DNA und deren
Verwendung als Arzneimittel

(iii) ANZAHL DER SEQUENZEN: 3

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30B (EPA)

(2) ANGABEN ZU SEQ ID NO: 1:

(i) SEQUENZKENNZEICHEN:
(A) LÄNGE: 917 Basenpaare
(B) ART: Nucleotid
(C) STRANGFORM: Einzelstrang
(D) TOPOLOGIE: linear

(ii) ART DES MOLEKÜLS: cDNA

(iii) HYPOTHETISCH: NEIN

(iv) ANTISENSE: NEIN

(vi) URSPRÜNGLICHE HERKUNFT:
(A) ORGANISMUS: PIG-F
(C) INDIVIDUUM/ISOLAT: Human

(xi) SEQUENZBESCHREIBUNG: SEQ ID NO: 1:

AGGAGGCAGT AGTTCCCCGC TTCCCTTCCG CGGGAGGGAG AGTTAGCTAG CCATCCAAGA 60

AAACACCATG AAAGATAACG ATATCAAGAG ACTACTGTAT ACCCATCTTT TATGCATATT 120

TTCAATTATC CTAAGTGTCT TCATTCCATC ACTCTTCTTG GAGAACTTCT CAATATTGGA 180

AACACACTTG ACATGGTTGT GCATCTGTTC TGGTTTTGTA ACTGCTGTCA ATCTAGTACT 240

ATATTTAGTA GTGAAACCAA ATACATCCTC TAAAAGAAGT TCATTATCAC ACAAGGTAAC 300

TGGATTTTTG AAATGCTGTA TCTACTTTCT TATGTCTTGT TTCTCCTTTC ATGTAATTTT 360

TGTTCTGTAT GGAGCACCAC TGATAGAGTT GGCATTGGAA ACATTTTTAT TTGCAGTTAT 420

TTTGTCTACT TTTACTACTG TGCCTTGCTT ATGTTTGTTA GGACCAAACC TCAAAGCATG 480

GCTAAGAGTG TTCAGTAGAA ATGGAGTTAC ATCCATATGG GAGAATAGTC TCCAGATCAC 540

TACAATTTCT AGCTTTGTAG GAGCATGGCT TGGAGCACTT CCTATTCCAC TGGATTGGGA 600

AAGACCATGG CAGGTATGGC CCATCTCCTG TACGCTTGGA GCGACCTTTG GCTACGTGGC 660

TGGCCTTGTT ATTTCACCAC TCTGGATATA CTGGAATAGA AAGCAACTTA CATACAAGAA 720

CAATTAACTG GAGCAAAGGG AGATATTTCT TTGTGCAGAT TCTGTAAGGG CTGGGCAGAA 780

ATGTGTATGG TCAAAGCCAA GCAGTTCCAT TTACAGCTCT GTTTTTTACG TAGTTACAAC 840

ATGATGTGAT TGTAGCTTTT TAAACTATGA AACCCCTGAG AGATTGTACC TTCTAGTTGA 900

AATAAAGTAT TTATAAT 917

(2) ANGABEN ZU SEQ ID NO: 2:

(i) SEQUENZKENNZEICHEN:
(A) LÄNGE: 219 Aminosäuren
(B) ART: Aminosäure
(C) STRANGFORM: Einzelstrang
(D) TOPOLOGIE: linear

(ii) ART DES MOLEKÜLS: Protein

(iii) HYPOTHETISCH: NEIN

(iv) ANTISENSE: NEIN

(vi) URSPRÜNGLICHE HERKUNFT:
(A) ORGANISMUS: PIG-F
(C) INDIVIDUUM/ISOLAT: Human

(xi) SEQUENZBESCHREIBUNG: SEQ ID NO: 2:

Met Lys Asp Asn Asp Ile Lys Arg Leu Leu Tyr Thr His Leu Leu Cys

1               5                   10                  15

Ile Phe Ser Ile Ile Leu Ser Val Phe Ile Pro Ser Leu Phe Leu Glu

            20                  25                  30

Asn Phe Ser Ile Leu Glu Thr His Leu Thr Trp Leu Cys Ile Cys Ser

        35                  40                  45

Gly Phe Val Thr Ala Val Asn Leu Val Leu Tyr Leu Val Val Lys Pro
    50                  55                  60
Asn Thr Ser Ser Lys Arg Ser Ser Leu Ser His Lys Val Thr Gly Phe
65                  70                  75                  80
Leu Lys Cys Cys Ile Tyr Phe Leu Met Ser Cys Phe Ser Phe His Val
                85                  90                  95
Ile Phe Val Leu Tyr Gly Ala Pro Leu Ile Glu Leu Ala Leu Glu Thr
            100                 105                 110
Phe Leu Phe Ala Val Ile Leu Ser Thr Phe Thr Thr Val Pro Cys Leu
        115                 120                 125
Cys Leu Leu Gly Pro Asn Leu Lys Ala Trp Leu Arg Val Phe Ser Arg
    130                 135                 140
Asn Gly Val Thr Ser Ile Trp Glu Asn Ser Leu Gln Ile Thr Thr Ile
145                 150                 155                 160
Ser Ser Phe Val Gly Ala Trp Leu Gly Ala Leu Pro Ile Pro Leu Asp
                165                 170                 175
Trp Glu Arg Pro Trp Gln Val Trp Pro Ile Ser Cys Thr Leu Gly Ala
            180                 185                 190
Thr Phe Gly Tyr Val Ala Gly Leu Val Ile Ser Pro Leu Trp Ile Tyr
        195                 200                 205
Trp Asn Arg Lys Gln Leu Thr Tyr Lys Asn Asn
    210                 215

(2) ANGABEN ZU SEQ ID NO: 3:

(i) SEQUENZKENNZEICHEN:
(A) LÄNGE: 2071 Basenpaare
(B) ART: Nucleotid
(C) STRANGFORM: Einzelstrang
(D) TOPOLOGIE: linear

(ii) ART DES MOLEKÜLS: cDNA

(iii) HYPOTHETISCH: NEIN

(iv) ANTISENSE: NEIN

(v) ART DES FRAGMENTS: linear

(vi) URSPRÜNGLICHE HERKUNFT:
(A) ORGANISMUS: RES c-DNA
(C) INDIVIDUUM/ISOLAT: Human

(xi) SEQUENZBESCHREIBUNG: SEQ ID NO: 3:

GGGATCAAGA TGTATAAACT GTTGTTTAAT TACGTGAGAA ACATCTTCAG TGGCCAAGGA 60

AACTGTCCAT TTCTCTCAGA AAGCAAGTGA AATGCTACAG CTATACCCAG ACCTTTTATA 120

GGTAATGAAG CAGTTCAAAA CTTGAAAGAA AACAAAACCT GTCCTCAGAA TTCTATAAAG 180

TGTATTAAGA ATGTTCCTTA AAGGTTTAAG AAGCAGTAAG CAGCATCTGA AGCCACAATC 240

TATTATAAAT ACTTTATTTC AACTAGAAGG TACAATCTCT CAGGGGTTTC ATAGTTTAAA 300

AAGCTACAAT CACATCATGT TGTAACTACG TAAAAAACAG AGCTGTAAAT GGAACTGCTT 360

GGCTTTGACC ATACACATTT CTGCCCAGCC CTTACAGAAT CTGCACAAAG AAATATCTCC 420

CTTTGCTCCA GTTAATTGTT CTTGTATGTA AGTTGCTTTC TATTCCAGTA TATCCAGAGT 480

GGTGAAATAA CAAGGCCAGC CACGTAGCCA AAGGTCGCTC CAAGCGTACA GGAGATGGGC 540

CATACCTGAG GAGAGAATGT ATGAGATCAA AAAAGAACAA ATGTTTTATT ATTACTTGAG 600

CACAAGTGTA ACCTAAATAT TTCTATATTA AAGCTTAATG TGCTTTCTTA AAGAATGCCA 660

AAAGTGTAAT AAGGTCATAA CTGCATTTAT CATGAACACT AAAAATGTAC ACATTTTAGT 720

TAATGTGCAT TAAACTGTAA CAAGGCTTCT GGCAATTGTA GATTTAGTTT GACGCTCCCC 780

AAAGTGCATG AGACACATGC TAAAATTACA AATTAAAATT TTGGGTCAGA CTTTGCCATA 840

ATGATAGACT CAATTTAGCT CTCTGAACTA GTTGGTAATT TTTTTTTTTT AATTCCCACT 900

TTGGCTGTGT ACATCAAATG AAATGAGAAG TGTGTTTGCT GACCAAACCA CAAGAAACTT 960

TCTTTAAGTT GTGTTAAAGA GGAAAGACCT AGAATCCAAG CGTGTTACAT GAAAATTGTA 1020

ACAGAGCAGC TGCTTCCACC TTTCAGATAT AGATGTTGGA ACCACAGCAG AAGTTATAGA 1080

GCGACAACTT ATATACACAC CTAGAATGTA AGTTAAACAA AATACCGGCT TCCAGAGACC 1140

CCTTTTCTCC AGCCATATTA CATCAGGCTA GAAGTAATTA ATGTTGATTT ATTTCATCTA 1200

CAAGCAGTTG GTCCCTAAGT GAAAGGCTCT GCTTGAAAAA AAAAAGAAAA AAAAGTTGGA 1260

GGAAAATTTT CATGTTCTTC TGTGAAGCTT ATTTGGTACA CTGGAGCCAT TTCTAATCTT 1320

TCTCTGGGGG GAACAGGCCA CAGAACTGTG TTAGAGGTGA ACCATCTTAA TTACTAGTTC 1380

TATTACCTAA TTCAGCTTCC TTGTTTGGTC TGCTGTGGAT CTGCCTTATT GCATATGCCA 1440

TGCATCAGAT AATGGATGCA TCAGATAATG GTGTTAGACA AAGCTTCATT GTGAACAACC 1500

TAATGCATTT TAGAGAAACA ATCTCATCAC ATTTTTTCTA GCCTTTCCTA CATTTAAACT 1560

TGCTGTTGCC CAAATTATAA TTTTTTAAAT GTCTTTGGTG GGCTTCTGTT AATTCACATG 1620

ACTTGAGCTT ATAGCTATGT CTACTGCACA GATTGGGTAA TGGAACACTA AACTTTTATA 1680

CTTGAAAATG ACAGCCTTAA ATGCTCATAT CAGTCACAAA TCTAGGATGT ACTGTCTTGT 1740

TGTATGTGAG CTTCGTAGAG ATTTTTAAAA ATATAAGCAT CACCTTCCCA TTGAAGAGTG 1800

GAGAGAGTCT ACTGGATGAC TGGCCAGGAA CTCCTTCTCT CTGAATCGGA CATTTGGATG 1860

TCTTCTCTTT CTTCCAAGAA ATGGTGGTTC ACATTAAAGA ATCATGGCCT TATGTATGCT 1920

CAAATGGAAT CTTATGTAAC TTTCTTATTT AATTTTGGTC TGCTTATTTT TAGATAAAAT 1980

TGAAAGAATT GTATAAATCA ATTAACATAT TAGCTGAGTT GTCCAACACA TGGTATAAAC 2040

GAATTACAAC AGTAAACTAT TACACATTTC C 2071

## Beschreibung

Die Annahme genetischer Schäden als Ursache der Entstehung von Tumoren wurde durch die Entdeckung von zellulären Genen, "Proto-Onkogene" genannt, bestätigt, die in genetisch modifizierter Weise als sogenannte "Onkogene" das neoplastische Wachstum bedingen. Trotz der genetischen Vielfalt der Proto-Onkogene können ihre biochemische Wirkungsmechanismen in wenige Kategorien zusammengefaßt werden. Ungefähr ein Drittel aller bisher bekannten Onkogene kodiert für Kinasen, die in Proteinen die Aminosäure Tyrosin phosphorylieren und die daher als Protein-Tyrosin-Kinasen (PTKs) bezeichnet werden. Viele Onkogene, die für Tyrosin-Kinasen kodieren, wurden zuerst im Genom von onkogenen Retroviren entdeckt. So stellt beispielsweise das im Rous-Sarkom-Virus vorkommende v-src Gen eine Modifikation des zellulären Proto-Onkogens dar. Die Besonderheit von v-src besteht darin, daß es als einziges Onkogen in der Lage ist, primäre Zellen ohne Mitwirkung eines zweiten Onkogens zu transformieren und auch in Hühnern Bindegewebstumoren hervorzurufen. V-src besitzt die Fähigkeit, Zellen unterschiedlichster Spezies zu transformieren. Diese außergewöhnlichen Eigenschaften sind darauf zurückzuführen, daß es sich bei v-src insofern um ein ganz außergewöhnliches Onkogen handelt als es mit einem besonders starken transformierenden Potential ausgestattet ist. Tumorsuppressorgene, die das onkogene Potential von v-src unterdrücken können, sind folglich als sehr stark einzuordnen.

### Reversion der v-src bedingten Transformation durch Expression der Okayama-Berg-Genbank

Die Zellen der RRC6-Zellinie, für die durch die Experimente der somatischen Zellhybridisierung gezeigt werden konnte, daß ihr transformierter Phänotyp revertierbar ist, wurden mit der cDNA humaner Vorhautfibroblasten, die in das pcd2-Expressionsplasmid kloniert sind (Okayama-Berg-Expressionsgenbank), transfiziert (OKAYAMA und BERG, 1982). Aus der genomischen DNA von Genbank-Transfektanten, die in ihrer Morphologie revertiert sind (Revertanten), können transformationssupprimierende cDNA-Inserts integrierter, rekombinanter pcd2-Plasmide isoliert werden. Die Reversion des transformierten Phänotyps dieser Revertanten kann mit der heterologen Transkription des bzw. der cDNA-Inserts der in die zelluläre RRC6-DNA integrierten pcd2-Plasmide korreliert werden. Revertanten sind nach der Definition von NODA Zellen einer Population transformierter Zellen, die durch das Wiederauftreten des nichtmalignen Phänotyps characterisiert sind (NODA, 1993).

### RRC6-Zellinie

Adhärent wachsende Zellen eines monoklonalen Subklons der RR1022-Zellen. Die Zellen dieser Zellinie haben fünf Kopien des v-src-Gens von RSV in ihre zelluläre DNA integriert und exprimieren die virale Kinase konstitutiv. Die enzymatische Aktivität von pp60^{v-src} beträgt 180 fmol übertragenes, anorganisches Phosphat pro mg Zelllysat-Protein in 3,5 Minuten bei 0°C in Gegenwart von Mn²⁺-Ionen.

### Hela-Karzinomzellinie (ATCC CCl-2: HAY et al., 1992)

Zellinie aneuploider, epithelzellähnlicher Zellen, die aus einem humanen Zervix-Adenokarzinom etabliert wurden. Die Zellen exprimieren Teile des humanen Papillomavirus (HPV-Typ 16)-Genoms. HPV-Typ 16 gehört zur Gruppe der "high risk"-HPV-Tumorviren.

Übertaschenderweise gelang bei der Transfektion der Okayama-Berg-Genbank in die transformierten RRC6-Zellen, verschiedene Revertantenklone zu isolieren, die in ihrer Morphologie und in ihrem Wachstumsverhalten normalen Zellen gleichen, beispielsweise durch die Expression eines bestimmten Plasmids mit einem cDNA-Insert von 2,4 kb (im folgenden Res2,4-cDNA genannt) konnte die v-src-bedingte Transformation komplett unterdrückt werden. Die Sequenz des transformationssupprimierenden Res2,4-cDNA-Inserts ist über eine Länge von 306 Nukleotiden identisch zur humanen PIG F-cDNA. Die Orientierung dieser natürlich vorkommenden Sequenzen ist gegenläufig (Expression in antisense-Richtung). Der Bereich der komplementären Sequenz des PIG F-Transkriptes umfaßt 193 Nukleotide seines 3' untranslatierten Bereichs und 113 Nukleotide seines offenen Leserahmens. Überraschenderweise konnte gezeigt werden, daß auch die Sequenz des gesamten offenen Leserahmens des humanen PIG F-Gens (in antisense-Orientierung in einen eukaryontischen Expressionsvektor kloniert) als heterologes Transkrist in den PRC6-Zellen transformationssupprimierendes Potential besitzt: Das durch PCR erzeugte PIG F-Amplifikat beinhaltet sowohl die zur effizienten Translation notwendigen Kozak-Konsensusmotive als auch das Polyadenylierungssignal dieser humanen cDNA. Aus der RNA Sup T1-Zellinie wurde mittels geeigneter Primer in 37 Zyklen des Taq-Syntheseschritts das PIG F-Transkript als 858 bp langes Fragment amplifiziert und in das pRC/CMV-Plasmid kloniert. Das pRC PIGₛ-Plasmid trägt die amplifizierte Sequenz der PIG F-cDNA in sense-Orientierung, das pRCPIGₐₛ-Plasmid trägt sie in antisense-Orientierung. Durch den CMV-Promotor des pRC/CMV Plasmids, unter dessen Kontrolle die PIG F-Sequenz in sense- und antisense-Orientierung kloniert wurden, erfolgt die konstitutive Transkription des jeweiligen Inserts in den rekombinanten eukaryontischen Zellen. Die pRCPIGₐₛ-Transfektanten sind in ihren morphologischen und zellbiologischen Eigenschaften (morphologische Reversion des transformierten Phänotyps, verminderte Fähigkeit der Zellen, in Weichagar Kolonien zu bilden) verändert und transkribieren das klonierte cDNA-Insert des transfizierten Plasmids. Die transformationsrelevante Kinaseaktivität des viralen pp60^{v-src}-Onkogenprodukts ist in diesen Zellen, die in ihrem transformierten Phänotyp revertiert sind, nachweisbar.

Die heterologe Transkription der sense-orientierten PIG F-cDNA-Sequenz des pRCPIGₛ-Plasmids hat in den Transfektanten keine Veränderungen der morphologischen und zellbiologischen Eigenschaften der rekombinanten Zellen zur Folge. Der transformierte Phänotyp dieser Zellen ist nicht revertiert.

Das heterologe Transkript der antisense-orientierten, kodierenden Sequenz des humanen PIG F-cDNA besitzt, wie auch die Sequenz des Res2,4-cDNA-Inserts, die partiell komplementär zur PIG F-cDNA ist, in den Zellen der rekombinanten RRC6-Zellinien transformationssupprimierendes Potential.

Aus diesen zusammengefaßten Ergebnissen wird deutlich, daß nur die heterologe Transkription des PIGₐₛ-cDNA-Inserts, das zur zellulären PIG F-mRNA komplementär ist, den transformierten Phänotyp der Zellen supprimieren kann. Dieses Transkript kann mit dem in den Zellen der RRC6-Zellen vorliegenden PIG F-Transkripft RNA-RNA-Hybride (Duplices) ausbilden. Diese Duplices werden die veränderte PIG F-Proteinkonzentration in diesen Zellen zur Folge haben, wodurch die Synthese des GPI-Ankers behindert wird. Hieraus ergibt sich die Beeinflussung der Zahl GPI-modifizierter Proteine auf der extrazellulären Seite der Plasmamembran. Die Modulation der Präsentation GPI-verankerter Proteine auf der Zellmembran kann demzufolge mit der Reversion des transformierten Phänotyps PIGₐₛ-Transfektanten korrelliert werden.

### Untersuchung der in vivo-Tumorigenizität revertierter Zellen in Nacktmäusen

Nachdem gezeigt werden konnte, daß die Reversion des transformierten Phänotyps in vitro durch die heterologe Transkription des zur zellulären PIG F-cDNA antisense-orientierten Inserts des pRC PIGₐₛ-Plasmids verursacht wird, wurden in vivo-Untersuchungen durchgeführt.

### Tumorigenizitätstest

Zur Bestimmung ihrer Tumorigenizität wurden die Zellen der RRC6-, der 208F-, der RpCMV D4- (Transfektanten des parentalen Expressionsplasmids pRC/CMV) und der RPIGₐₛ E9F1-Zellinie (Transfektanten der antisense-orientierten PIG F-cDNA) in jeweils fünf Nacktmäuse injiziert. Die Zellen der RRC6-Zellinie sind durch eine ausgeprägte Tumorigenizität in Nacktmäusen gekannzeichnet. Alle fünf Mäuse, denen auf der linken Seite 5x10⁵ Zellen der pp60^{v-src}-transformierten Rattenzellinie subkutan injiziert wurden, bilden an den Injektionsstellen große, schnell wachsende Tumore aus, von denen der größte Tumor die Maße 13 mm x 26 mm besitzt. Bereits 5x10³ Zellen reichen aus, die in die andere Flanke der thymusaplastische Maus subkutan injiziert werden, am 11. Tag nach der Injektion Tumore entstehen zu lassen, die ein progredientes Wachstum zeigen. Von den 5 Mäusen, die mit dieser Zellkonzentration auf der rechten Flanke belegt wurden, bilden vier einen Tumor aus. Die Maus, die nach 21 Tagen auf der rechten Flanke keinen Tumor ausbildete, bildet auch auf der rechten Seite nur einen relativ kleinen Tumor (5 mm x 5 mm), der deutlich kleiner ist als die auf der rechten Seite ausgebildeten Tumore der anderen Mäuse dieser Versuchsgruppe; möglicherweise eine Versuchstier-bedingte Schwankung.

Die Mäuse, denen die immortalisierten Rattenfibroblasten der 208F-Zellinie in den entsprechenden Zellkonzentrationen in die beiden Seiten injiziert wurden, bilden alle keinen Tumor aus. Obwohl auf der linken Seite des dritten Versuchstiers am 14. Tag nach Injektion ein 2 mm x 2 mm großes Geschwur tastbar gewesen ist, konnte am 18. Tag an dieser Stelle kein Tumor festgestellt werden.

Werden fünf Nacktmäusen die 5x10⁵ Zellen der RpCMV B8-Zellinie subkutan in die linke Flanke injiziert, entstehen an den Injektionsstellen aller fünf Mäuse Tumore. Das Wachstum dieser Tumore ist allerdings im Vergleich zu denen der durch die RRC6-Zellen induzierten Tumore weniger progressiv, d.h. sie wachsen weniger schnell. Auf jeder linken Flanke der Versuchstiere dieser Gruppe ist ein Tumor meßbar, doch nehmen die Tumore in ihrer Größe nicht so schnell zu, wie es die Tumore tun, die durch die pp60^{v-src}-transformierten Rattenzellen induziert werden. Diese Transfektanten des parentalen Plasmids pRC/CMV sind durch die Transfektion in ihrer Tumorigenizität an Nacktmäusen beeinflußt. Die durch diese Zellen induzierten Tumore wachsen langsamer als die RRC6-Tumore. Dieses Ergebnis wird auch dadurch unterstützt, daß von den fünf Mäusen, die mit 5x10³ Zellen dieser Zellinie auf der rechten Seite inokuliert wurden, nur eine Maus ein Tumor ausbildet. Die anderen Mäuse bilden nach 21Tagen keinen Tumor auf der rechten Seite aus.

Die monoklonale Zellpopulation der RPIGₐₛ E9F1-Zellinie erzeugt in den Nacktmäusen, die in den Konzentrationen von 5x10⁵ bzw. 5x10³ Zellen in die linke bzw. rechte Flanke injiziert wurden, keinen Tumor. Die Tumorigenizität dieser Zellen ist im Vergleich zu der der pp60^{v-src}-transformierten Zellen der RRC6-Zellinie signifikant reduziert. Die Reduktion der Tumorigenizität dieser rekombinanten RRC6-Zellen bestätigt die Ergebnisse des in vitro-Untersuchungen zur Bestimmung der Weichagarklonierungseffizienz. In diesen Versuchen konnte gezeigt werden, daß die Zellen dieser Zellinie durch die deutliche Reduktion ihrer Fähigkeit, in Weichagar Kolonien zu bilden, charakterisiert sind.

Durch die Versuche zur Tumorigenizität kann trotz der begrenzten Anzähl der Versuchstiere und der daraus resultierenden begrenzten Anzahl der untersuchten Zellinien eindeutig die Suppression des transformierten Phänotyps der Transfektanten in vivo gezeigt werden, die mit der in antisense-klonierten cDNA des humanen PIG F-Gens transfiziert wurden.

### Suppression des transformierten Phänotyps von Hela-Zellen

Zur weiteren Charakterisierung des transformationssupprimierenden Potentials der PIG F-cDNA wurden die Zellen der humanen Hela-Zervixkarzinomzellinie mit der DNA des Res2,4-Plasmids transfiziert und Revertanten isoliert, deren transformierter Phänotyp überraschenderweise, wie es bereits bei v-src transformierten Zellen der Fall war, ebenfalls morphologisch revertiert war. Dieses Ergebnis ist deswegen überraschend, weil der Hela Zelle ein anderer Transformationsmechanismus zugrundeliegt als der v-svc transformierten RRC6-Zelle. Vier Zellinien wurden etabliert. Drei dieser Zellinien sind durch veränderte morphologische und zellbiologische Eigenschaften charakterisiert. In den rekombinanten Zellen dieser Hela-Zellinien wird die heterologe Transkription des Res2,4-cDNA-Inserts durch die RNA-PCR eindeutig nachgewiesen.

Die Zellen der HRes2,4 A9-Transfektantenzellinie sind ihrer Morphologie nur partiell revertiert. Trotz der partiell veränderten Zellgestalt zeigten die Zellen keine verminderte Fähigkeit, in Weichagar Kolonien zu bilden. Die heterologe Transkription des Res2,4-cDNA-Inserts kann in den Zellen nicht nachgewiesen werden. Diese Zellpopulation belegt deshalb deutlich, daß die Reversion des transformierten Phänotyps nur durch die Transkription der klonierten cDNA des Res2,4-Plasmids verursacht wird. Durch die heterologe Transkription der klonierten cDNA kann die Bildung von Duplexmolekülen erfolgen, die aus den über 306 Nukleotiden komplementären mRNA Molekülen des zellulären PIG F und des heterologen cDNA-Transkripts gebildet werden können. Daraus resultierend wird vermutlich die Synthese des GPI-Ankers beeinflußt. Durch die dadurch veränderte Präsentation GPI-verankerter Proteine auf der extrazellulären Seite der Plasmamembran könnte ursächlich die Reversion des durch das humane Papillonsvirus 16 bedingte transformierten Phänotyps der rekombinanten Zellen resultieren.

Dieses Experiment ist ein Beleg dafür, daß die Expression der PIG F-antisense-RNA zum einen die Tranformation von Fibroblasten durch die Protein-Tyrosin-Kinase v-src als auch von Cervix-Zellen durch die Papillomvirusgene E6/E7 unterdrücken kann. Damit kann der PIG F-antisense RNA ein allgemein supprimierender Effekt zugeschrieben werden.

### GPI-verankerte Proteine

In eukaryontischen Zellen wurden zahlreiche Oberflächenantigene identifiziert, die mittels des GPI-Ankers posttranslational an ihrem C-Terminus modifiziert werden, und auf der extrazellulären Seite der Plasmamembran zu finden sind: In der Ratte bzw. der Maus werden die Oberflächenproteine Thy-1; Rt-6.2 Qa-2 als Proteine der extrazellulären Seite der Plasmamembran präsentiert (ROBINSON et al., 1989, KOCH et al., 1986). Für die humanen Oberflächenantigene CD14 (Makrophagen-Differenzierungsantigen), CD16 (FCgIII-Rezeptor), CD24, CD29, CD48, CD55 (Decay accelerating factor-DAF), CD59, CD67, CD73 (5'-Nukleotidase), Ly-6.1 und Ly-6.2 (T-Zelloberflächenmarker) wurde die posttranslationale Modifikation durch den GPI-Anker nachgewiesen. Beispiele für GPI-verankerte Enzyme sind z.B. CD73, Acetylcholinesterase der Erythrozyten und Alkalische Phosphatase (TURNER und HOOPER, 1990). Weiterhin wurde gezeigt, daß einige Liganden der eph/elk-RPTK-Familie durch die GPI-Verankerung auf der extrazellulären Seite spezieller Zellen präsentiert werden (HOLZMAN et al., 1990; BARTLEY et al., 1994). Aber auch das mit den Liganden der FGF-Rezeptorfamilie wechselwirkende Heparinproteoglukan (HPG) wird ebenfalls auf der Plasmamembran präsentiert. Die essentielle Wechselwirkung des HPG mit Liganden der FGF-R erfolgt nach Abspaltung des HPG durch die spezielle Phosphatidylinositol-Phospholipase D (METZ et al., 1994).

Allen GPI-verankerten Proteinen ist die C-terminale Modifikation durch einen GPI-Anker gemeinsam. Dieser GPI-Anker besteht aus einer zentralen Kernstruktur, die z.T. durch Substituenten stark modifiziert wird. Aus der Substitution zusätzlicher Zuckermoleküle oder Fettsäuren an diesen Anker resultiert vermutlich die Spezifität der entsprechenden Proteine (CROSS, 1990). Aufgrund der gemeinsamen Kernstruktur des GPI-Ankers der präsentierten Proteine ist davon auszugehen, daß dessen Synthese mittels eines evolutionär konservierten Synthesewegs gebildet wird (CARAS und MORAN, 1994). Der Syntheseweg des GPI-Ankers und die Identifizierung kodierender Sequenzen der am Syntheseweg beteiligten Enzyme ist Gegenstand umfangreicher Untersuchungen, um dadurch weitere Hinweise auf die Funktion GPI-verankerte Proteine zu erhalten. Bis heute konnten drei cDNAs isoliert werden, deren kodierte Proteine an der GPI-Synthese in eukaryontischen Zellen beteiligt sind:
Phosphatidyl-Inositol-Glukan Klasse A: PIG A (MIYATA et al., 1993)
Phosphatidyl-Isositol-Glukan Klasse B: PIG B (KINOSHITA et al., 1994)
Phsophatidyl-Inositol-Glukan Klasse F: PIG F (INUOR et al., 1993).

Da die Hemmung der GPI-Synthese durch Einsatz von PIG F-antisense RNA zur Hemmung von Krebszellen führt, läßt sich überraschenderweise die Schlußfolgerung aufstellen, daß GPI-verankerte Oberflächenproteine an der Aufrechterhaltung des transformierten Phänotyps beteiligt sind, indem sie den Differenzierungs- und Proliferationszustand von Zellen beeinflussen.

Zur Bekämpfung und Heilung von Tumoren können folgende Wege eingeschlagen werden:

### Gentherapeutische Methoden:

Hierzu werden geeignete Vektoren (nach dem jeweiligen Stand der Technik) in den Tumorzellen exprimiert, die Teilsequenzen der PIG F-mRNA als antisense-cDNA-Konstrukte tragen. Aufgrund der oben beschriebenen Versuchsergebnisse sollen folgende Sequenzen zur Hemmung zellulärer Teilungsaktivität exprimiert werden:
a) der gesamte offene Leseraster von PIG F in antisense-Orientierung (siehe Sequenzlisting)
b) Teilsequenzen des kodierenden Bereichs von PIG F
b₁) 3'-Bereich der kodierenden Region: 306 Bp bestehend aus 113 Bp des 3' Endes des offenen Leserasters zuzüglich 193 Bp des 3'untranslatierten Bereichs
b₂) Fragmente von PIG F, die den gesamten Leseraster in Schritten von 100 Bp abdecken.

Ferner können synthetische Oligonucleotide oder Oligonucleotid-Analoga, die Teilen von PIG F entsprechen, eingesetzt werden.

## Patentansprüche

1. PIG-F c-DNA mit der Sequenz ii) No. 1 und deren funktionellen Derivate und Fragmente.

2. Vektor enthaltend die DNA gemäß Anspruch 1.

3. Arzneimittel enthaltend die DNA gemäß Anspruch 1.

4. RES-cDNA mit der Sequenz ii) No. 2 und deren funktionellen Derivate und Fragmente.

5. Vektoren enthaltend die DNA gemäß Anspruch 4.

6. Arzneimittel enthaltend die DNA gemäß Anspruch 4.

7. Arzneimittel, die die Synthese des GPI-Antens blockieren.

8. Natürliche und synthetische Antisensenukleotide zu der DNA gemäß Anspruch 1 und 4.
